# EUROPÄISCHE PATENTANMELDUNG

(11) **EP 2 065 988 A2**
(43) Veröffentlichungstag der Anmeldung: **03.06.2009**
(21) Anmeldenummer: 08167226.3
(22) Anmeldetag: 22.10.2008
(51) Int. Cl.: H01R 43/10

(54) **Verfahren und Vorrichtung zur Reparatur eines Schleifringes im eingebauten Zustand**

(30) Priorität: 20.11.2007 US 989252 P; 03.03.2008 DE 102008000489
(71) Anmelder: Schleifring und Apparatebau GmbH, 82256 Fürstenfeldbruck (DE)
(72) Erfinder: Staffler, Herbert, 82256 Fürstenfeldbruck (DE); Suchanecki, Ronald, 81679 München (DE); Schilling, Harry, 85072 Eichstätt (DE); Schünke, Fabian, 82256 Fürstenfeldbruck (DE)
(74) Vertreter: Lohr, Georg

(57) **Zusammenfassung**

Die Erfindung betrifft ein Verfahren sowie eine Vorrichtung zur Durchführung des Verfahrens zur Bearbeitung der Schleifbahnen von Schleifringen in einem in eine Maschine eingebauten Zustand. Hierzu wird der Schleifring durch den Antrieb der Maschine gedreht, während ein Werkzeug, dass von einer Werkzeughaltevorrichtung gehalten wird, über die Schleifbahnen geführt wird, so dass das Material von der Oberfläche der Schleifbahnen abgetragen wird.

## Beschreibung

### Technisches Gebiet

Die Erfindung bezieht sich auf eine Vorrichtung sowie ein Verfahren zur Reparatur beziehungsweise Überarbeitung der Oberfläche von Schleifringen, wobei die Schleifringe in ihrem eingebauten Zustand in einem Gerät verbleiben.

### Stand der Technik

Schleifringe werden üblicherweise in einer mechanischen Fertigung hergestellt und dann in ein Gerät eingebaut. Ein solches Gerät kann beispielsweise eine größere Maschine wie die Gantry eines CT-Scanners (Computertomograph) sein. Im Betrieb des Schleifrings entsteht ein mechanischer Verschleiß durch die mechanische Reibung der Bürste auf der Schleifbahn. Bei Schleifringen mit Kohlebürsten auf Metallbahnen ist der Abrieb an denen Kohlebürsten selbst wesentlich größer als der Abrieb an dem Schleifring. Die Kohlebürsten sind so gestaltet, dass sie relativ einfach ausgetauscht werden können. Bei längerem Betrieb macht sich jedoch auch ein deutlicher Verschleiß des Schleifrings selbst bemerkbar. So können in der Oberfläche eines Schleifrings beziehungsweise seiner Schleifbahnen Riefen, Rillen und andere Vertiefungen entstehen. Diese Beschädigungen der Oberfläche führen nun zu einem erhöhten Kontaktwiderstand und zu einem erhöhten Verschleiß der Kohlebürsten. Ähnliches gilt für den Fall von Schleifringen mit Metallbürsten. So kann auch hier ein Verschleiß der Oberfläche des Schleifrings nach längerem Betrieb festgestellt werden. Die Beschädigungen der Oberfläche können abhängig von den Betriebsbedingungen nach einigen Monaten bis mehreren Jahren auftreten. Grundsätzlich gilt, dass die Bürsten relativ einfach austauschbar sind, während eine Reparatur des Schleifrings verhältnismäßig aufwändig ist. So wird nach dem Stand der Technik ein Schleifring zur Reparatur aus dem Gerät ausgebaut und in einer Werkstatt oder auch in der Fertigung des Herstellers überarbeitet oder aber auch durch einen neuen Schleifring ersetzt. Dies hat den Nachteil, dass das Gerät längere Zeit still steht und ein relativ Zeit- und arbeitsaufwändiger Austausch des Schleifrings vorgenommen werden muss. Insbesondere CT-Scanner müssen zum Austausch des Schleifrings weitgehend zerlegt werden. Da gerade diese Schleifringe, wie beispielsweise in der US 4,782,580 A offenbart, Innendurchmesser in einer Größenordnung von 1,5 m und mehr haben, können diese auch nur mittels spezieller großer Drehbänke überarbeitet werden.

### Beschreibung der Erfindung

Der Erfindung liegt die Aufgabe zugrunde, ein Verfahren zur Reparatur von Beschädigungen einer Schleifring-Oberfläche, insbesondere verschlissener Schleifring-Bahnen vorzustellen, welches den zur Reparatur bisher benötigten Zeitaufwand sowie die notwendigen Kosten reduziert. Weiterhin soll die Erfindung eine Vorrichtung zur Durchführung des Verfahrens angegeben.

Eine erfindungsgemäße Lösung dieser Aufgabe ist in den unabhängigen Patentansprüchen angegeben. Weiterbildungen der Erfindung sind Gegenstand der abhängigen Ansprüche.

In dem erfindungsgemäßen Verfahren wird der Schleifring nicht mehr aus dem Gerät ausgebaut, sondern in diesem selbst bearbeitet. Hierzu wird an dem Gerät ein Werkzeugträger befestigt, der ein Werkzeug zur Bearbeitung der Oberfläche des Schleifrings, vorzugsweise der Schleifbahnen hält. Ein solches Werkzeug kann beispielsweise ein Material abtragendes oder auch ein die Oberfläche verformendes Werkzeug sein. Material abtragende Werkzeuge sind beispielsweise Schneidstähle zur spanabhebenden Bearbeitung oder auch Schleifwerkzeuge. Die Oberfläche verformende Werkzeuge können beispielsweise Rollen zum Rollieren sein. Grundsätzlich sind auch andere Arten von Werkzeugen zur Bearbeitung der Oberfläche der Schleifbahnen möglich. Wesentlich ist hierbei jedoch, dass die Oberfläche der zu bearbeitenden Schleifbahnen eingeebnet beziehungsweise geglättet wird. Wahlweise kann ein einziges Werkzeug oder auch eine Mehrzahl von Werkzeugen gleichzeitig oder nacheinander eingesetzt werden. So könnten beispielsweise mit einer Mehrzahl von Werkzeugen mehrere Schleifbahnen gleichzeitig bearbeitet werden. Alternativ können auch unterschiedliche Werkzeuge zeitlich beziehungsweise räumlich hintereinander eingesetzt werden.

Um die Schleifbahnen entlang ihrer gesamten Länge bearbeiten zu können, wird der Schleifring relativ gegenüber dem Werkzeug bewegt. Der Schleifring selbst bleibt hierbei weiterhin fest mit dem Gerät verbunden. Die Relativbewegung erfolgt daher vorzugsweise durch einen Antrieb des Geräts mit dem ein mit dem Schleifring verbundener Teil des Gerätes gedreht wird. Relevant ist hierbei eine Drehbewegung um die Drehachse des Schleifrings. So kann beispielsweise beim Einsatz in einem Computertomographen der Motor zum Antrieb des drehenden Teils der Gantry hierzu verwendet werden. Vorzugsweise weist der Antrieb zur Durchführung der Oberflächenbearbeitung des Schleifrings beziehungsweise der Schleifbahnen einen geeigneten Betriebsmodus, wie beispielsweise eine besonders geringe Geschwindigkeit auf. So können durch eine niedrige Geschwindigkeit mechanische Vibrationen des Gerätes geringer gehalten und die Oberflächenqualität verbessert werden. Für den Fall, dass es sich um eine im Normalbetriebsfall extrem langsam drehende Maschine handelt, kann der Antrieb auch eine Betriebsart mit höherer Geschwindigkeit zur Bearbeitung der Schleifbahnen aufweisen.

Mit dem erfindungsgemäßen Verfahren kann der Schleifring wesentlich besser an das Gerät angepasst werden, als dies in einer Fertigungsanlage, getrennt von dem Gerät möglich ist. So wird beispielsweise der Rundlauf eines Schleifrings nicht nur durch die mechanischen Toleranzen des Gerätes sowie des Schleifrings selbst bestimmt. Er wird auch beeinflusst durch die Präzision der Montage des Schleifrings in dem Gerät. Wird nun der Schleifring entsprechend dem erfindungsgemäßen Verfahren in dem Gerät überarbeitet, so können die Rundlauf Toleranzen unter Eliminierung aller dieser Einflüsse minimiert werden. Typischerweise ist eine Verbesserung der Rundlauftoleranzen (Radialschlag) um den Faktor 10 möglich.

In einer vorteilhaften Ausgestaltung der Erfindung ist der Antrieb des Gerätes in verschiedenen (Dreh-) Geschwindigkeiten steuerbar, um jeweils optimale Bedingungen für die Bearbeitung der Oberfläche herzustellen. So kann beispielsweise durch geeignete Software auch ein spezielles Geschwindigkeitsprofil, beispielsweise mit langsamem Anfahren bis zur Bearbeitungsgeschwindigkeit und langsamem Abbremsen nach der Bearbeitung gesteuert werden. Erfindungsgemäß wird die Geschwindigkeit entsprechend den Anforderungen durch die Oberflächebearbeitung eingestellt. Es wird hier ausdrücklich auf eine Relativbewegung zwischen Schleifring und Werkzeug Bezug genommen. Typischerweise wird das Werkzeug in einer festen Position wahlweise am Gerät befestigt oder aber auch mit einem Ständer nahe am Gerät aufgestellt. Die Befestigung an dem Gerät kann beispielsweise mit Schrauben, Bolzen oder einer Schnellspannvorrichtung erfolgen. Während der Bearbeitung wird der Schleifring gedreht. Alternativ ist es aber auch möglich, während der Bearbeitung den Schleifring fest stehen zu lassen und das Werkzeug zu drehen. Ebenso könnten aber auch Schleifring und Werkzeug gleichzeitig, aber mit unterschiedlichen Geschwindigkeiten gedreht werden.

In einer weiteren vorteilhaften Ausgestaltung der Erfindung wird die zur Bearbeitung der Oberfläche des Schleifrings notwendige relativen Bewegung zwischen dem Werkzeug und dem Schleifring mit einem Hilfsantrieb erzeugt. Dieser Hilfsantrieb kann wahlweise fest in das Gerät integriert sein. Alternativ kann er aber auch vor Beginn der Bearbeitung der Oberfläche an dem Gerät befestigt und nach Weise mit Schrauben, Bolzen oder einer Schnellspannvorrichtung an dem Gerät befestigt und nach Abschluss der Bearbeitung wieder von dem Gerät entfernt werden. Dies ist besonders günstig, wenn der Antrieb des Gerätes selbst nicht in einem Geschwindigkeitsbereich arbeiten kann der für die Überarbeitung des Schleifrings geeignet ist. Vorzugsweise ist an dem Gerät eine Aufnahme für den Hilfsantrieb vorgesehen, so das der Hilfsantrieb auf einfache Weise mit Schrauben, Bolzen oder einer Schnellspannvorrichtung an dem Gerät befestigt werden kann. Wahlweise kann der Hilfsantrieb auch mit dem Werkzeugträger in eine Einheit integriert sein. Auch der Hilfsantrieb wird vorzugsweise in seiner Geschwindigkeit beziehungsweise in seinem Geschwindigkeitsprofil wie zuvor beschrieben gesteuert.

In der einfachsten Ausführungsform der Erfindung erfolgt die Steuerung beziehungsweise die Positionierung des Werkzeugs manuell durch einen Bediener des Geräts. Dieser Bediener kann dann das Werkzeug in eine günstige Bearbeitungsposition zur Bearbeitung der Oberfläche der einzelnen Schleifbahnen bringen. Weiterhin kann der Bediener dann das Werkzeug während des gesamten Bearbeitungsvorganges entsprechend über der Oberfläche des Schleifrings beziehungsweise der einzelnen Schleifbahnen führen. Optional ist ein halbautomatischer Betrieb realisierbar, in dem der Bediener nur das Werkzeug auf die Schleifbahn ausrichtet und dann automatisch eine Steuerung durch eine Steuereinheit des Werkzeugs an der Schleifbahn erfolgt. Im Falle eines Drehstahls kann beispielsweise durch die Steuereinheit eine sukzessive Zustellung über die gesamte Schleifbahn erfolgen, bis diese vollständig überdreht ist. Für einen halbautomatischen Betrieb ist eine einfache elektrische oder auch mechanische Steuereinheit, beispielsweise eine einfache Vorschubsteuerung ausreichend.

In einer weiteren Ausgestaltung der Erfindung erfolgt die Steuerung der Position des Werkzeugs mittels des Werkzeugträgers durch einen Stellantrieb. Hiermit ist eine besonders einfache und meist auch schnelle Positionierung des Werkzeugs möglich. Vorteilhafterweise erfolgt die Positionierung programmgesteuert nach einem vordefinierten Ablaufschema.

In einer weiteren Ausgestaltung der Erfindung ist eine gemeinsame Steuerung des Positionierantriebs des Werkzeugträgers sowie des Antriebs des Schleifrings vorgesehen. Dadurch kann beispielsweise zuerst das Werkzeug in eine geeignete Bearbeitungsposition und dann die Drehzahl des Schleifrings auf eine geeignete Bearbeitungsgeschwindigkeit gebracht werden.

In einer weiteren vorteilhaften Ausgestaltung der Erfindung erfolgt die Steuerung des Werkzeugs mittels einer Steuereinheit, die die Geometriedaten des Schleifrings in einem Speicher bereithält. Alternativ können durch die Steuereinheit die Geometriedaten auch aus dem Gerät, in welches der Schleifring eingebaut ist abgerufen werden. Vorteilhafterweise wird aus dem Gerät jedoch nur eine Kennung des eingebauten Schleifrings abgerufen. Alternativ hierzu könnte die Steuereinheit auch aus dem Gerät eine Geräteerkennung abrufen und aus einer Datenbank die Kennung sowie die Geometriedaten des eingebauten Schleifrings abfragen. In einer weiteren Ausgestaltung kann die Steuereinheit auch durch weitere Abfragemittel, wie Barcode, Transponder etc. den Schleifring identifizieren. Alternativ könnte auch eine Schleifring- oder Gerätekennung durch einen Bediener eingegeben werden. Die Steuereinheit kann wahlweise eine externe Steuereinheit sein, die erst zur Überarbeitung des Schleifrings mit dem Werkzeugträger bereitgestellt wird. Alternativ hierzu kann die Steuereinheit auch bereits in das Gerät, beispielsweise einen Computertomographen integriert sein. So kann einer der zahlreichen Mikrocontroller und Rechner, welche in einem Gerät wie beispielsweise einen Computertomographen ohnehin vorhanden sind, auch die Aufgabe einer Steuereinheit übernehmen. Zu diesem Zweck ist lediglich eine gesonderte Software sowie geeignete Schnittstellen zur Steuerung des Werkzeugträgers zu integrieren.

In einer weiteren vorteilhaften Ausgestaltung ist wenigstens ein Sensor zur Erkennung wenigstens einer Schleifbahn vorgesehen. Vorteilhafterweise ist dieser Sensor in die Steuereinheit integriert oder zumindest mit dieser verbunden. Damit wird die Steuereinheit in die Lage versetzt, die Position einer oder mehrerer Schleifbahnen zu erkennen und das Werkzeug entsprechend einzustellen. Grundsätzlich ist hiermit auch ein vollautomatischer Betrieb möglich. So kann wahlweise bei bekannten Geometriedaten des Schleifrings durch zusätzliche Sensoren die Bearbeitungsgenauigkeit erhöht werden. Ebenso kann eine hochwertige Bearbeitung auch ohne bekannte Geometriedaten erreicht werden.

Es ist besonders vorteilhaft, wenn zumindest ein Sensor zur Erfassung der Qualität der Oberfläche einer Schleifbahn vorhanden ist. Ein solcher Sensor kann beispielsweise den Kontaktwiderstand oder auch die Rauhigkeit der Oberfläche einer Schleifbahn ermitteln. Es ist auch möglich, beliebige mechanische Parameter wie Rundlauf, Bandbreite, Bahntiefe oder auch Höhe und/oder Breite der Isolationsstege zwischen den Schleifbahnen zu ermitteln. Die von dem wenigstens einen Sensor registrierten Werte können an die Steuereinheit zur Steuerung der Bearbeitung signalisiert werden. So kann beispielsweise die Schleifbahn so weit bearbeitet werden, bis ein minimaler vorgegebener Übergangswiderstand erreicht ist, oder bis eine bestimmte Oberflächenbeschaffenheit, beispielsweise Rauhtiefe erreicht ist. Weiterhin kann auch wahlweise ein Sensor zur Ermittlung weiterer Parameter vorgesehen sein. Dieser kann beispielsweise die Isolationsstege zwischen Schleifbahnen prüfen und Abweichungen an die Steuereinheit übermittelt. Vorteilhafterweise umfasst ein solcher Sensor eine Kamera.

Die im normalen Betriebsfall auf den Schleifbahnen laufenden Bürsten können während der Überarbeitung von den Schleifbahnen abgehoben werden. Dies trägt insbesondere zur Schonung der Bürsten bei. So entsteht kein zusätzlicher Verschleiß der Bürsten durch kurzfristig während der Bearbeitung auftretende Unebenheiten der Schleifbahn. Bei Bearbeitungsverfahren wie Rollieren oder Schleifen, bei denen kontinuierlich die Rauhigkeit der Oberfläche verringert wird, ist es auch möglich, die Bürsten an der Schleifbahn anliegen zu lassen. Vorteilhafterweise wird in diesem Falle mittels der Bürsten der Übergangswiderstand zwischen Bürsten und Schleifbahn ermittelt. Dieser kann dann zur weiteren Steuerung der Bearbeitung verwendet werden. So kann beispielsweise die Bearbeitung bei unterschreiten eines Widerstands-Grenzwertes beendet werden.

In einer weiteren vorteilhaften Ausgestaltung der Erfindung erfolgt die Bearbeitung in wenigstens zwei Schritten mit wenigstens zwei unterschiedlichen Werkzeugen. Dies können beispielsweise unterschiedliche Schneidstähle oder auch in einem ersten Schritt ein Schneidstahl und in einem zweiten Schritt ein Schleifwerkzeug sein. Ebenso kann beispielsweise in einem ersten Schritt ein grober Abtrag der Oberfläche mittels eines Schneidstahls und im zweiten Schritt eine Glättung mittels eines Rollierwerkzeugs erfolgen.

Insbesondere bei Computertomographen kann der rotierende Teil der Gantry häufig gekippt werden. So kann beispielsweise zur Bearbeitung der Schleifbahnen eines Schleifrings auch dieser gekippt werden, um eine gewisse Vorspannung des Gantry- Lagers und hiermit eine spezielle Lage des Schleifrings zu erreichen. Weiterhin kann hierdurch auch eine vereinfachte Entfernung des Abriebs beziehungsweise der Späne während der Bearbeitung erreicht werden. Besonders günstig ist es jedoch, den Schleifring in seiner normalen Betriebsposition zu belassen.

Eine erfindungsgemäße Vorrichtung zur Durchführung des zuvor beschriebenen Verfahrens umfasst einen Werkzeugträger zur Aufnahme eines Werkzeugs zur Bearbeitung der Oberfläche des Schleifrings, vorzugsweise wenigstens einer Schleifbahn. Bevorzugt umfasst die Vorrichtung wenigstens ein entsprechendes Werkzeug zur Bearbeitung der Oberfläche des Schleifrings beziehungsweise wenigstens einer Schleifbahn. Vorteilhafterweise weist der Werkzeugträger wenigstens einen elektrisch oder pneumatisch gesteuerten Stellantrieb zur Positionierung des Werkzeugs auf. Weiterhin kann optional eine Steuereinheit zur Steuerung des Stellantriebs vorgesehen sein.

In einer besonders vorteilhaften Ausführungsform der erfindungsgemäßen Vorrichtung ist noch ein Hilfsantrieb zur Bewegung des Schleifrings gegenüber dem Werkzeugträger vorgesehen. Ein solcher Hilfsantrieb kann vorteilhafterweise, wie bereits zuvor beschrieben, an dem Gerät befestigt werden. Bevorzugt ist an dem Gerät eine Aufnahme hierzu vorgesehen.

Ein erfindungsgemäßes Gerät, insbesondere ein Computertomograph umfasst eine Vorrichtung zur Aufnahme einer zuvor beschriebenen Vorrichtung, insbesondere eines Werkzeugträgers. Optional umfasst das Gerät eine zusätzliche Aufnahme für einen Hilfsantrieb. Weiterhin umfasst es optional eine Steuereinheit zur Steuerung der Bearbeitung. In einer weiteren vorteilhaften Ausgestaltung umfasst es einen Speicher, in dem Identifikationsdaten zur Identifikation des Schleifrings und/oder Geometriedaten des Schleifrings gespeichert sind.

Der Begriff des Gerätes, in den der Schleifring eingebaut ist, bezieht sich auf ein Gerät, in dem der Schleifring auch tatsächlich zu Übertragung mindestens eines elektrischen Signals eingesetzt wird. Nicht gemeint ist hiermit eine Aufspannung eines Schleifrings im einer Werkzeugmaschine, wie beispielsweise einer Drehbank.

In den vorhergehenden Ausführungen wird auf die Bearbeitung der Oberfläche eines Schleifrings Bezug genommen. Grundsätzlich kann jedoch mit einem erfindungsgemäßen Verfahren sowie einer erfindungsgemäßen Vorrichtung jeder beliebige Bereich der Oberfläche eines Schleifrings bearbeitet werden. Somit kann auch beispielsweise das Trägermaterial oder auch das Isolationsmaterial bearbeitet werden. Dies macht beispielsweise dann Sinn, wenn die Oberfläche des Isolationsmaterial durch starke Funkenbildung beschädigt worden ist. Auch könnten hier zu einem Upgrade des Schleifrings zusätzliche Nuten für die Aufnahme neuer Schleifbahnen eingebracht werden. Da mit dem erfindungsgemäßen Verfahren Bereiche an der Oberfläche eines Schleifrings hergestellt werden können, die minimale Rundlauftoleranzen aufweisen, kann dies auch gezielt in der Fertigung eingesetzt werden. So kann die Auflagefläche für die Sendeantenne einer kontaktlosen Hochgeschwindigkeits-Datenstrecke zuerst nach dem erfindungsgemäßen Verfahren bearbeitet werden, so dass diese einen minimalen Radialschlag oder Axialschlag je nach Montage in Richtung der Antenne aufweist. Nach der Bearbeitung kann dann die Antenne aufgebracht werden. Weiterhin kann im Feldeinsatz durch das erfindungsgemäße Verfahren auch eine defekte Antenne von dem Träger des Schleifrings entfernt, beispielsweise abgedreht oder abgeschliffen werden.

Der bevorzugte Einsatzbereich des erfindungsgemäßen Verfahrens sowie der erfindungsgemäßen Vorrichtung ist jedoch die Überarbeitung, insbesondere zur Reparatur der Schleifbahnen.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.

Fig. 1 zeigt eine erste erfindungsgemäße Anordnung.

Fig. 2 zeigt einen Computertomographen.

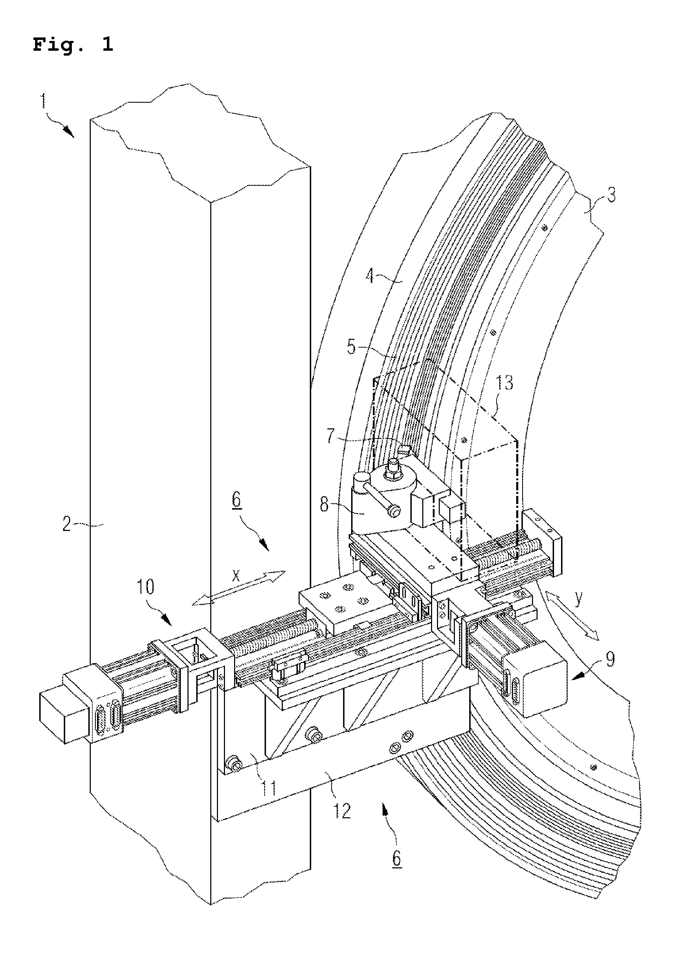

In Figur 1 ist schematisch ein Beispiel eines Werkzeugträgers mit Werkzeug entsprechend der Erfindung dargestellt. Die später ausführlicher erläuterte Gantry 1 eines Computertomographen umfasst ein stationäres Teil 2 sowie ein rotierendes Teil 3. Der Schleifring 4 ist an dem rotierenden Teil 3 befestigt. An der Oberfläche des Schleifrings 4 sind die Schleifbahnen 5 angeordnet. Die erfindungsgemäße Vorrichtung umfasst einen Werkzeugträger, der mittels eines Befestigungselementes 11 an dem Befestigungselement 12 des stationären Teils 2 der Gantry 1 befestigt ist. Zur Bearbeitung der Oberfläche des Schleifrings 4 hält der Werkzeugträger 6 ein Werkzeug 7. In diesem Beispiel ist das Werkzeug 7 ein Schneidstahl. Dieser wird in einer Schnellspannvorrichtung 8 aufgenommen. Das Werkzeug 7 ist gegenüber der Oberfläche des Schleifrings 4 axial und radial beweglich. Zur Positionierung des Werkzeugs 7 in axialer Richtung Y ist der Stellantrieb 9 vorgesehen. Ein weiterer Stellenantrieb 10 ermöglicht die Positionierung des Werkzeugs in radialer Richtung X bezogen auf das rotierende Teil der Gantry und somit auch auf den Schleifring. Beide Stellenantriebe haben hier beispielhaft einen Antriebsmotor, der eine Spindel zur Bewegung der Schlitten dreht. Bevorzugterweise sind auch noch Positionssensoren, wie beispielsweise lineare Wegencoder oder auch Winkelencoder vorgesehen, um die exakte Position des Schlitten und somit des Werkzeugs zu erfassen. In dem hier gezeigten Ausführungsbeispiel ist zunächst der Stellantrieb 10 auf dem Befestigungselement 11 angeordnet. Auf dem Schlitten dieses Stellantriebs ist dann der zweite Stellantrieb 9 befestigt. Es wird hier also der komplette zweite Stellantrieb in radialer Richtung X bewegt. Weiterhin ist auf dem Schlitten des zweiten Stellantriebs 9 das Werkzeug 7 mittels einer Schnellspannvorrichtung 8 befestigt. Durch die einfache Art der Befestigung des gesamten Werkzeugträgers mittels des Befestigungselementes 11 an dem Befestigungselement 12 des stationären Teils der Gantry kann der Werkzeugträger beispielsweise durch Lösen der hier gezeigten Schrauben auf einfache Art und Weise entfernt werden. Vorteilhafterweise sind an dem Befestigungselement 11 und/oder an dem Befestigungselement 12 noch zusätzliche Positionierhilfen, wie Anschläge, Stifte oder anderes zur exakten Ausrichtung und Justierung der beiden Befestigungselemente und somit auch des Werkzeugträgers 6 gegenüber der Gantry 1 vorgesehen.

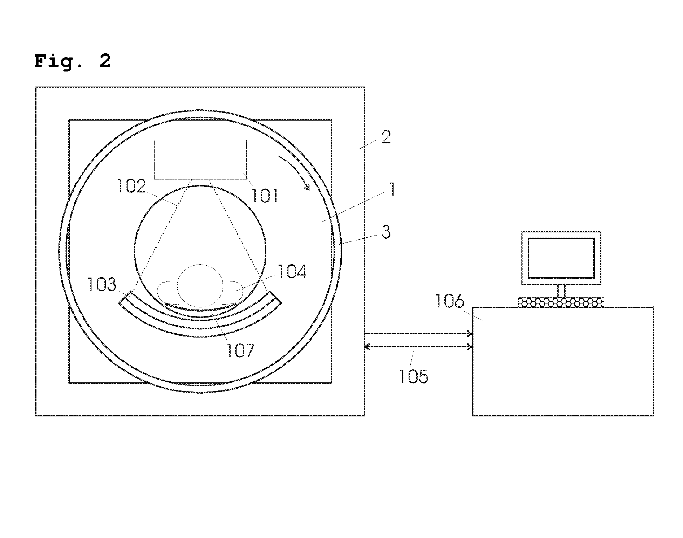

Figur 2 zeigt eine erfindungsgemäße Vorrichtung am Beispiel eines Computertomographen. Der Computertomograph (CT-Scanner) besteht aus zwei mechanischen Hauptbestandteilen. Ein stationäres Teil 2 dient als Basis und Träger des ganzen Gerätes, in denen sich das rotierende Teil 1 dreht. Der Patient 104 wird auf einer Liege 107 in der Öffnung des rotierenden Teils positioniert. Zur Abtastung des Patienten mittels Röntgenstrahlen 102 ist eine Röntgenröhre 101 sowie ein dieser gegenüberliegend angeordneter Detektor 103 vorgesehen. Röntgenröhre 101 und Detektor 103 sind auf dem rotierenden Teil 1 drehbar angeordnet. Ein Drehübertrager 3 dient zur elektrischen Verbindung zwischen dem rotierenden Teil 1 und dem stationären Teil 2. Hierbei werden einerseits die hohe elektrische Leistung zur Speisung der Röntgenröhre 101 in Richtung des rotierenden Teils 1 und gleichzeitig die Rohdaten des Bildes in der entgegengesetzten Richtung übertragen. Parallel hierzu ist eine Kommunikation von Steuerinformationen in beiden Richtungen vorgesehen. Eine Auswerte- und Steuereinheit 106 dient zur Bedienung des Computertomographen sowie zur Anzeige der erzeugten Bilder. Die Kommunikation mit dem Computertomographen erfolgt über eine bidirektionale Verbindung 105.

### Bezugszeichenliste

- 1: Gantry eines Computertomographen
- 2: Stationäres Teil der Gantry
- 3: Rotierendes Teil der Gantry
- 4: Schleifring
- 5: Schleifbahnen
- 6: Werkzeugträger
- 7: Werkzeug
- 8: Schnellspannvorrichtung für Werkzeug
- 9: Stellantrieb zur Positionierung in axialer Richtung
- 10: Stellantrieb zur Positionierung in radialer Richtung
- 11: Befestigungselement des Werkzeugträgers
- 12: Befestigungselement am stationären Teil der Gantry
- 13: Sammelbehälter
- 101: Röntgenröhre
- 102: Röntgenstrahlung
- 103: Detektor
- 104: Patient
- 105: bidirektionale Verbindung
- 106: Auswerte- und Steuereinheit
- 107: Patientenliege

## Patentansprüche

1. Verfahren zur Bearbeitung der Oberfläche eines Schleifrings (4) welcher in einem Gerät (1) eingebaut ist mit folgenden, zeitlich aufeinanderfolgenden Verfahrensschritten:
- Anbringen eines Werkzeugträgers (6) mit einem Werkzeug (7) an dem Gerät (1)
- Drehen des Schleifrings (4) gegenüber dem Werkzeugträger (6)
- Bearbeiten der Oberfläche des Schleifrings (4) mit dem Werkzeug (7).

2. Verfahren nach Anspruch 1,
**gekennzeichnet durch**
Bearbeiten der Oberfläche des Schleifrings (4) mit einem spanabhebenden Werkzeug (7), einem schleifenden Werkzeug oder einem die Oberfläche verformenden Werkzeug.

3. Verfahren nach einem der vorhergehenden Ansprüche,
**gekennzeichnet durch**
Drehen des Schleifrings (4) mit einem Antrieb des Gerätes (1) oder mit einem Hilfsantrieb.

4. Verfahren nach einem der vorhergehenden Ansprüche,
**gekennzeichnet durch**
Steuern der Drehgeschwindigkeit des Schleifrings (4) zur Anpassung an den Bearbeitungsvorgang.

5. Verfahren nach einem der vorhergehenden Ansprüche,
**gekennzeichnet durch**
Steuern der Position des Werkzeuges (7) mittels einer Steuereinheit.

6. Verfahren nach einem der vorhergehenden Ansprüche,
**gekennzeichnet durch**
Steuern der Position des Werkzeuges (7) unter Berücksichtigung von in einem Speicher abgelegten Geometriedaten des Schleifrings (4).

7. Verfahren nach einem der vorhergehenden Ansprüche,
**gekennzeichnet durch**
Steuern der Position des Werkzeuges (7) unter Erkennung der Position und/oder der Abmessungen wenigstens einer zu bearbeitenden Schleifbahn mittels wenigstens eines Sensors.

8. Verfahren nach einem der vorhergehenden Ansprüche,
**gekennzeichnet durch**
Messen des Übergangswiderstandes der gerade bearbeiteten Schleifbahn mittels der für den normalen Betrieb vorgesehenen Schleifbürsten und Abbruch des Bearbeitens der Schleifbahn, nachdem der Übergangswiderstand einen vorgegebenen Grenzwert unterschritten hat.

9. Vorrichtung zur Bearbeitung der Oberfläche eines Schleifrings (4) welcher in ein Gerät (1) eingebaut ist, nach einem Verfahren entsprechend einem der vorhergehenden Ansprüche umfassend einen Werkzeugträger (6) zur Aufnahme wenigstens eines Werkzeuges (7) zur Bearbeitung der Oberfläche des Schleifrings (4), wobei die Vorrichtung an dem Gerät (1) angebracht werden kann.

10. Vorrichtung nach Anspruch 9 umfassend ein Werkzeug (7) zur Bearbeitung der Oberfläche eines Schleifrings (4), insbesondere wenigstens einer Schleifbahn.

11. Vorrichtung nach Anspruch 9 oder 10,
**dadurch gekennzeichnet dass,**
wenigstens ein elektrisch oder pneumatische gesteuerter Stellantrieb (9,10) zur Positionierung des Werkzeugs (7) vorgesehen ist.

12. Vorrichtung nach Anspruch 11,
**dadurch gekennzeichnet dass,**
eine Steuereinheit zur Steuerung des wenigstens einen Stellantriebs (9,10) vorgesehen ist.

13. Vorrichtung nach einem der Ansprüche 9 bis 12,
**dadurch gekennzeichnet dass,**
ein Hilfsantrieb zur Drehung des Schleifrings (4) relativ zum Werkzeug (7) während der Bearbeitung vorgesehen ist, welcher an dem Gerät (1) angebracht werden kann.

14. Gerät (1), insbesondere Computertomograph umfassend wenigstens eine Aufnahme (11) zur Befestigung wenigstens einer Vorrichtung (6) zur Bearbeitung der Oberfläche eines Schleifrings (4) nach einem der Ansprüche 9 bis 13.

15. Gerät (1) nach Anspruch 14,
**dadurch gekennzeichnet dass,**
der Antrieb des Geräts (1) zur Drehung des Schleifrings (4) während der Durchführung des Verfahrens nach einem der Ansprüche 1 bis 9 ausgelegt ist.
